# EUROPEAN PATENT APPLICATION

(11) **EP 4 545 091 A1**
(43) Date of publication of application: **30.04.2025**
(21) Application number: 23206072.3
(22) Date of filing: 26.10.2023
(51) Int. Cl.: A61K 38/57, A61P 29/00, A61P 31/14, A61P 35/00, C07K 14/81

(54) **TRANSMEMBRANE SERINE PROTEASE AND NEUTROPHIL ELASTASE INHIBITORS**

(71) Applicant: Universität Ulm, 89081 Ulm (DE)
(72) Inventor: MÜNCH Jan, 89081 Ulm (DE); WETTSTEIN, Lukas, 89081 Ulm (DE); LAWRENZ, Jan, 89081 Ulm (DE); RODRIGUEZ-ALFONSO, Armando, 89081 Ulm (DE); PREISING, Nico, 89081 Ulm (DE); STÄNDKER, Ludger, 89081 Ulm (DE); WIESE, Sebastian, 89081 Ulm (DE)
(74) Representative: Altmann Stößel Dick Patentanwälte PartG mbB

(57) **Abstract**

The present invention relates to an inhibitory polypeptide for use in treating and/or preventing a transmembrane serine protease (TMPRSS) and/or neutrophil elastase mediated disease in a subject, wherein said inhibitory polypeptide comprises an amino acid sequence of SEQ ID NO:1 or an amino acid sequence at least 70% identical thereto. The present invention further relates to polynucleotides, host cells, devices, kits, and uses related thereto.

## Description

The present invention relates to an inhibitory polypeptide for use in treating and/or preventing a transmembrane serine protease (TMPRSS) and/or neutrophil elastase mediated disease in a subject, wherein said inhibitory polypeptide comprises an amino acid sequence of SEQ ID NO:1 or an amino acid sequence at least 70% identical thereto. The present invention further relates to polynucleotides, host cells, devices, kits, and uses related thereto.

Respiratory viruses, such as coronaviruses, influenza viruses, metapneumoviruses, and parainfluenza viruses, depend on proteolytic cleavage of proteins mediating infection of target cells. E.g. SARS-CoV-2 infects target cells via an interaction of the viral spike protein with the cellular receptor ACE2, which interaction requires a proteolytic activation of the spike protein by the cellular transmembrane serine protease 2 (TMPRSS2, cf. e.g. Böttcher et al. (2006) JVI 80(19):9896). However, also other proteases, e.g. TMPRSS11D, TMPRSS13, and neutrophil elastase were found to play important roles in respiratory virus infection (Belouzard et al. (2010) JBC 285(3):22758; Emboriadou et al. (2007) Ann Clin Lab Sci 37(1):79; Kishimoto et al. (2021) Viruses 13:384; Hoffmann et al. (2021) EbioMedicine 65:103255; Böttcher et al. (2006) loc. cit).

In accordance, protease inhibitors have been candidate compounds for inhibition of viral infection, cf. e.g. Hoffmann et al. (2020) Cell 181: 271-280.e8; Hoffmann et al. (2020), Antimicrob. Agents Chemother. 64: 19-21, and the compounds Paxlovid und Molnupiravir were approved for the treatment of severe SARS-CoV-2 infection. Also, Aprotinin was approved for treatment of influenza in Russia and was tested in a clinical phase III study on treatment of SARSCoV-2 infection (Redondo-Calvo et al. (2022) Eur J Clin Invest 52:e13776).

Moreover, proteases like TMPRSSs and neutrophil elastase were found to play a role in the pathogenesis of cancer, such as prostate cancer (Ko et al. (2020) Oncogene 39:5950) and in pancreatitis.

Nonetheless, there is a need in the art for improved means and methods for improved treatments of the aforesaid diseases and conditions. Thus, the technical problem underlying the present invention can be seen as the provision of means and methods for complying with the aforementioned needs. The technical problem is solved by the embodiments characterized in the claims and herein below.

In accordance, the present invention relates to an inhibitory polypeptide for use in treating and/or preventing a transmembrane serine protease (TMPRSS) and/or neutrophil elastase mediated disease in a subject, wherein said inhibitory polypeptide comprises an amino acid sequence of SEQ ID NO:1 or an amino acid sequence at least 70% identical thereto.

The present invention also relates to an inhibitory polypeptide for use in treating and/or preventing a respiratory virus infection in a subject, wherein said inhibitory polypeptide comprises an amino acid sequence of SEQ ID NO: 1 or an amino acid sequence at least 70% identical thereto.

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one". In accordance, expressions relating to one item of a plurality, unless otherwise indicated, preferably relate to at least one such item, more preferably a plurality thereof; thus, e.g. identifying "a cell" relates to identifying at least one cell, preferably to identifying a multitude of cells. Also, the term "plurality" relates to a multitude, preferably at least two, more preferably at least three, still more preferably at least four, of the indicated items.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

The methods specified herein below, preferably, are in vitro methods. The method steps may, in principle, be performed in any arbitrary sequence deemed suitable by the skilled person, but preferably are performed in the indicated sequence; also, one or more, preferably all, of said steps may be assisted or performed by automated equipment. Moreover, the methods may comprise steps in addition to those explicitly mentioned above.

As used herein, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1% by weight, most preferably less than 0.1% by weight of non-specified component(s).

The term "polynucleotide", as used herein, refers to a linear or circular nucleic acid molecule. The polynucleotide of the present invention shall be provided, preferably, either as an isolated polynucleotide, i.e. isolated from its natural context, or in genetically modified form, preferably comprising at least one heterologous sequence. The term polynucleotide encompasses single-as well as, partially or completely, double-stranded polynucleotides. Preferably, the polynucleotide is a DNA polynucleotide, which may also be referred to as "DNA". Moreover, comprised are also chemically modified polynucleotides including naturally occurring modified polynucleotides such as glycosylated or methylated polynucleotides or artificially modified derivatives such as biotinylated polynucleotides, locked nucleic acids, peptide nucleic acids, and the like.

Unless specifically indicated otherwise, reference to specific polynucleotides herein preferably includes polynucleotide variants. The term "polynucleotide variant", as used herein, relates to a variant of a polynucleotide referred to herein comprising a nucleic acid sequence characterized in that the sequence can be derived from the aforementioned specific nucleic acid sequence by at least one nucleotide substitution, addition and/or deletion, wherein the polynucleotide variant shall have the function and/or activity as specified for the specific polynucleotide. Thus, a variant of a polynucleotide may e.g. be an ortholog, a paralog, or another homolog of the specific polynucleotide; the polynucleotide variant may also be a mutant of the specific polynucleotide, preferably a naturally occurring mutant. Also preferably, said polynucleotide variant is or is derived from a non-naturally occurring allele of the specific polynucleotide. Further polynucleotide variants include polynucleotides comprising nucleic acid sequences which are at least 70%, preferably at least 80%, more preferably at least 90%, even more preferably at least 95%, still more preferably at least 98%, most preferably at least 99%, identical to the specifically indicated nucleic acid sequences. The percent identity values are, preferably, calculated over the entire nucleic acid sequence region, preferably as specified herein elsewhere. The polynucleotides of the present invention either consist of, essentially consist of, or comprise at least one of the aforementioned nucleic acid sequences. Thus, they may contain further nucleic acid sequences as well.

The term "protein" is understood by the skilled person; preferably, the protein comprises at least one amino acid chain, i.e. a polypeptide as specified herein below, more preferably comprises a multitude of polypeptides. Thus, the protein may be a multimer, e.g. a dimer, a trimer, or the like, wherein the polypeptides in the multimer may be connected covalently, e.g. by a disulfide bridge, or non-covalently, e.g. by ionic interactions, hydrophobic interactions, and/or van der Waals interactions. The protein may consist of identical polypeptides, e.g. may be a homodimer, or may comprise at least two nonidentical polypeptides, e.g. may be a heterodimer. More preferably, the protein as specified comprises all structural components as indicated comprised in one continuous covalent polypeptide chain, thus, the protein preferably is or is comprised in a fusion polypeptide.

The term "polypeptide", as used herein, refers to a molecule consisting of a multitude of amino acids that are covalently linked to each other by peptide bonds. Polypeptides consisting of less than 20 amino acids covalently linked by peptide bonds may also be referred to as "peptides". Preferably, the polypeptide comprises of from 25 to 1000, more preferably of from 40 to 250, still more preferably of from 50 to 100, most preferably about 51 amino acids. The polypeptide may also be comprised in a fusion polypeptide, i.e. may comprise amino acid sequences in addition to those specifically indicated. Also, the polypeptide may comprise additional, non-peptidic structures, such as at least one glycosylation, lipid conjugation, and the like. Thus, unless specifically indicated otherwise, reference to specific polypeptides herein preferably includes polypeptide variants.

As used herein, the term "polypeptide variant" relates to any chemical molecule comprising at least one polypeptide as specified herein differing in structure from a specifically indicated polypeptide. Preferably, the polypeptide variant, in particular the inhibitory polypeptide, comprises a polypeptide having a contiguous amino acid sequence corresponding to at least 70%, preferably at least 80%, more preferably at least 90%, even more preferably at least 95%, still more preferably at least 98%, most preferably at least 99%, of the amino acid sequence of the polypeptide specifically indicated. Moreover, it is to be understood that a polypeptide variant as referred to in accordance with the present invention shall have an amino acid sequence which differs due to at least one amino acid substitution, deletion and/or addition, wherein the amino acid sequence of the polypeptide variant, in particular the inhibitory polypeptide, is still, preferably, at least 70%, more preferably at least 80%, even more preferably at least 90%, even more preferably at least 95%, still more preferably at least 98%, most preferably at least 99%, identical with the amino acid sequence of the specific polypeptide. The degree of identity between two amino acid sequences can be determined by algorithms well known in the art and as described herein elsewhere. Polypeptide variants referred to above may be allelic variants or any other species specific homologs, paralogs, or orthologs. Moreover, the polypeptide variants referred to herein include fragments of the specific polypeptides or the aforementioned types of polypeptide variants, preferably as long as these fragments and/or variants have the activity as specified, e.g. immunogenicity in the case of epitopes or polypeptides comprising them. Such fragments may be or may be derived from, e.g., degradation products or splice variants of the polypeptides. Further included are variants which differ due to posttranslational modifications such as phosphorylation, glycosylation, ubiquitinylation, sumoylation, or myristylation, by including non-natural amino acids, and/or by being peptidomimetics. The "activity" of a polypeptide as specified herein is, preferably, preserved in the polypeptide variant; as the skilled person will understand in view of the description herein, the activity of a polypeptide does not necessarily have to reflect its main natural function, but may be a further activity relevant in the context of the claimed invention. The above applies to peptide variants and to protein variants mutatis mutandis.

The term "fragment" of a biological macromolecule, preferably of a polynucleotide or polypeptide, is used herein in a wide sense relating to any sub-part, preferably subdomain, of the respective biological macromolecule comprising the indicated sequence, structure and/or activity. Thus, the term includes sub-parts generated by actual fragmentation of a biological macromolecule, but also sub-parts derived from the respective biological macromolecule in an abstract manner, e.g. in silico. Thus, as used herein, an Fc or Fab fragment, but also e.g. a single-chain antibody, a bispecific antibody, and a nanobody may be referred to as fragments of an immunoglobulin. Also, a disease epitope may be a fragment of a disease antigen.

Unless specifically indicated otherwise herein, the compounds specified, in particular the polynucleotides and polypeptides, may be comprised in larger structures, e.g. may be covalently or non-covalently linked to further sequences, adjuvants, carrier molecules, retardants, and other excipients. In particular, polypeptides as specified may be comprised in fusion polypeptides comprising further polypeptides, which may serve e.g. as a tag for purification and/or detection, as a linker, or to extend the in vivo half-life of a compound. The term "detectable tag" refers to a stretch of amino acids which are added to or introduced into the fusion polypeptide; preferably, the tag is added C- or N- terminally to the fusion polypeptide. Said stretch of amino acids preferably allows for detection of the polypeptide by an antibody which specifically recognizes the tag; or it preferably allows for forming a functional conformation, such as a chelator; or it preferably allows for visualization, e.g. in the case of fluorescent tags. Preferred detectable tags are the Myc-tag, FLAG-tag, 6-His-tag, HA-tag, GST-tag or a fluorescent protein tag, e.g. a GFP-tag. These tags are all well known in the art. Other further peptides preferably comprised in a fusion polypeptide comprise further amino acids or other modifications which may serve as mediators of secretion, as mediators of blood-brain-barrier passage, as cell-penetrating peptides, and/or as immune stimulants. Further polypeptides or peptides to which the polypeptides may be fused are signal and/or transport sequences, e.g. an IL-2 signal sequence, and linker sequences.

The degree of identity (e.g. expressed as "%identity") between two biological sequences, preferably DNA, RNA, or amino acid sequences, can be determined by algorithms well known in the art. Preferably, the degree of identity is determined by comparing two optimally aligned sequences over a comparison window, where the fragment of sequence in the comparison window may comprise additions or deletions (e.g., gaps or overhangs) as compared to the sequence it is compared to for optimal alignment. The percentage is calculated by determining, preferably over the whole length of the polynucleotide or polypeptide as specified herein, the number of positions at which the identical residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (e.g. BLAST, GAP, BESTFIT, PASTA, or TFASTA), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. More preferably, the Basic Local Alignment Search Tool (BLAST) implementation is used with default parameter values for alignment. In the context of biological sequences referred to herein, the term "essentially identical" indicates a %identity value of at least 90%, preferably at least 95%, more preferably at least 98%, most preferably at least 99%. As will be understood, the term essentially identical includes 100% identity. The aforesaid applies to the term "essentially complementary" mutatis mutandis.

The term "transmembrane serine protease", which may be abbreviated as "TMPRSS", is known to the skilled person to relate to a family of type II transmembrane serine proteases. Preferably, the TMPRSS is a human TMPRSS. Preferably, the TMPRSS is TMPRSS2, TMPRSS11D, or TMPRSS 13. More preferably, the TMPRSS is human TMPRSS2, preferably human TMPRSS2 comprising the amino acid sequence of Genbank Acc No. NP_001128571.1, or is human TMPRSS11D, preferably human TMPRSS11D comprising the amino acid sequence of Genbank Acc No. NP_004253.1, or is human TMPRSS13, preferably human TMPRSS13 comprising the amino acid sequence of Genbank Acc No. NP_001070731.1.

The term "neutrophil elastase", relates to a serine protease from the chymotrypsin family of proteases. Preferably, the neutrophil elastase is a human neutrophil elastase, preferably comprising the amino acid sequence of Genbank Acc No. NP001963.1.

The term "inhibitory polypeptide", as used herein, relates to any polypeptide having the activity of inhibiting at least one TMPRSS and/or neutrophil elastase and comprising an amino acid sequence of SEQ ID NO:1 or an amino acid sequence at least 70%, preferably at least 80%, more preferably at least 90%, even more preferably at least 95%, still more preferably at least 98%, most preferably at least 99%, identical thereto. The inhibitory polypeptide preferably comprises, more preferably is, a trypstatin or a fragment thereof having the indicated activity. Thus, the inhibitory polypeptide preferably is a proteolytic fragment of an AMBP preprotein, preferably of a human AMBP preprotein comprising the amino acid sequence of Genbank Acc No. NP_001624.1. The inhibitory polypeptide preferably comprises an amino acid sequence of SEQ ID NO:1. Also preferably, the inhibitory polypeptide comprises, preferably consists of, an amino acid sequence of SEQ ID NO:1 or 2 or an amino acid sequence at least 70% identical thereto, more preferably the inhibitory polypeptide comprises, more preferably consists of, an amino acid sequence of SEQ ID NO:1 or 2.

The inhibitory polypeptide has the activity of inhibiting at least one TMPRSS and/or neutrophil elastase. In view of the description herein above, the inhibitory polypeptide preferably has the activity of inhibiting at least one protease selected from the group consisting of TMPRSS2, TMPRSS11D, TMPRSS13, and neutrophil elastase, preferably selected from the group consisting of human TMPRSS2, human TMPRSS11D, human TMPRSS13, and human neutrophil elastase. Methods for determining inhibition of at least one of the aforesaid protease by an inhibitory polypeptide are known in the art and are described herein in the Examples. Preferably, said inhibition is tested by incubating one of the proteases in vitro with a chromogenic substrate in the presence and absence of the inhibitory polypeptide; and/or is tested by infection of cells in vitro by e.g. SARS-CoV-2 or by luciferase-encoding lentiviral (pseudo-)particles harboring the SARS-CoV-2 spike protein in the presence and absence of the inhibitory polypeptide.

The inhibitory polypeptide may be prepared by any means deemed appropriate by the skilled person. Thus, the inhibitory polypeptide may be produced by chemical synthesis, e.g. in a protein synthesizer known in the art, or may be produced in an appropriate host cell. In view of the description herein, the skilled person understands that the inhibitory polypeptide may be produced as such, i.e. with the amino acid sequence as specified; the inhibitory polypeptide may, however, also be produced as a precursor molecule, e.g. as an AMBP preprotein, and optionally be subsequently proteolytic ally processed. Proteolytic processing may, however, be dispensable, since the precursor molecule may be proteolytically processed in the body of the subject. Thus, the inhibitory polypeptide may be administered as such, i.e. as a protein or polypeptide, but the inhibitory polypeptide may also be administered as a polynucleotide encoding the inhibitory polypeptide, e.g. as an mRNA, or as a host cell producing the inhibitory polypeptide. As the skilled person understands, it may be preferable to include measures ensuring that the inhibitory polypeptide is available on the outside of target cells; thus, e.g. in a host cell producing the inhibitory polypeptide, the inhibitory polypeptide may be produced as a precursor including a secretion sequence; appropriate sequences are known in the art.

The term "transmembrane serine protease (TMPRS) and/or neutrophil elastase mediated disease", as used herein, includes each and every disease in which a TMPRSS and/or neutrophil elastase contribute(s) to severity of disease and/or aggravation of its symptoms; corresponding diseases are known in the art and are discussed herein elsewhere. Also, it may be required by applicable regulations to test whether a TMPRSS and/or neutrophil elastase contribute(s) to severity of disease and/or aggravation of its symptoms before the use of an inhibitory polypeptide described herein in treatment and/or prevention of such disease, e.g. by standard clinical tests of clinical efficacy. Preferably, the TMPRSS and/or neutrophil elastase mediated disease is a disease in which the protease activity of said TMPRSS and/or neutrophil elastase contributes to aggravation of at least one symptom. Thus, the TMPRSS and/or neutrophil elastase mediated preferably disease is a virus infection, cancer, or pancreatitis, wherein said virus infection preferably is a respiratory virus infection.

In view of the above, the inhibitory polypeptide preferably is for use in treating and/or preventing a respiratory virus infection in a subject. The term "respiratory virus" is known to the skilled person. Preferably, the term relates to a virus infecting the respiratory tract of a subject, in particular the nasopharynx, larynx, trachea, bronchi, and/or lung tissue, and in particular mucous membranes thereof. Respiratory viruses are known in the art, as has been the requirement of said viruses for TPMRSs and/or neutrophil elastase for effective entry. In accordance, the respiratory virus preferably is a coronavirus (CoV), an influenza A virus, an influenza B virus, a human metapneumovirus, or a human parainfluenza virus, wherein the coronavirus preferably is a severe acquired respiratory syndrome (SARS) virus, preferably SARS-CoV-2, a Middle East respiratory syndrome-related (MERS)-CoV, a hCoV-229E, a hCoV-NL63, a hCoV-OC43, or a hCoV-HKU1; the aforesaid viruses and virus strains are known to the skilled person. In view of the above, the term "respiratory virus infection" relates to any infection, preferably of the respiratory tract, with at least one of the aforesaid respiratory viruses. Thus, the respiratory virus infection preferably is a coronavirus infection, an influenza A infection, an influenza B infection, a human metapneumovirus infection, or a human parainfluenza virus infection.

The term "cancer", as used herein, relates to a disease of an animal, including man, characterized by uncontrolled growth by a group of body cells ("cancer cells"). This uncontrolled growth may be accompanied by intrusion into and destruction of surrounding tissue (infiltration) and possibly spread of cancer cells to other locations in the body (metastasis). Preferably, also included by the term cancer is a recurrence of a cancer (relapse). Thus, preferably, the cancer is a solid cancer, a metastasis, or a relapse thereof. Preferably, the cancer is selected from the list consisting of acute myeloid leukemia (AML), acute lymphoblastic leukemia, adrenocortical carcinoma, aids-related lymphoma, anal cancer, appendix cancer, astrocytoma, atypical teratoid, basal cell carcinoma, bile duct cancer, bladder cancer, brain stem glioma, breast cancer, burkitt lymphoma, carcinoid tumor, cerebellar astrocytoma, cervical cancer, chordoma, chronic lymphocytic leukemia, chronic myelogenous leukemia, colon cancer, colorectal cancer, craniopharyngioma, endometrial cancer, ependymoblastoma, ependymoma, esophageal cancer, extracranial germ cell tumor, extragonadal germ cell tumor, extrahepatic bile duct cancer, fibrosarcoma, gallbladder cancer, gastric cancer, gastrointestinal stromal tumor, gestational trophoblastic tumor, hairy cell leukemia, head and neck cancer, hepatocellular cancer, hodgkin lymphoma, hypopharyngeal cancer, hypothalamic and visual pathway glioma, intraocular melanoma, kaposi sarcoma, laryngeal cancer, medulloblastoma, medulloepithelioma, melanoma, merkel cell carcinoma, mesothelioma, mouth cancer, multiple endocrine neoplasia syndrome, multiple myeloma, mycosis fungoides, nasal cavity and paranasal sinus cancer, nasopharyngeal cancer, neuroblastoma, non-hodgkin lymphoma, non-small cell lung cancer, oral cancer, oropharyngeal cancer, osteosarcoma, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pheochromocytoma, pituitary tumor, pleuropulmonary blastoma, primary central nervous system lymphoma, prostate cancer, rectal cancer, renal cell cancer, retinoblastoma, rhabdomyosarcoma, salivary gland cancer, sézary syndrome, small cell lung cancer, small intestine cancer, soft tissue sarcoma, squamous cell carcinoma, squamous neck cancer, testicular cancer, throat cancer, thymic carcinoma, thymoma, thyroid cancer, urethral cancer, uterine sarcoma, vaginal cancer, vulvar cancer, waldenström macroglobulinemia, and wilms tumor. Preferably, the cancer is a cancer overexpressing at least one TMPRSS and/or neutrophil elastase and/or secreting at least one TMPRSS and/or neutrophil elastase. Thus, the cancer preferably is prostate cancer.

The term "pancreatitis" is known to the skilled person to relate to any inflammation of the pancreas. Preferably pancreatitis is chronic or acute pancreatitis.

The terms "treating" and "treatment" refer to an amelioration of a disease or disorder referred to herein or the symptoms accompanied therewith to a significant extent; as used herein, the term includes prevention of deterioration of a disease, disorder, or symptoms associated therewith. Said treating may also include an entire restoration of health with respect to the diseases or disorders referred to herein. It is to be understood that treating, as the term is used herein, may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 10%, at least 20% at least 50% at least 60%, at least 70%, at least 80%, or at least 90% of the subjects of a given cohort or population. In treatment of virus infection, treatment preferably comprises inhibition of virus entry into target cells and, thus, inhibition of cell-to-cell spread of virus particles. As the skilled person is aware of, effectiveness of treatment of virus infection may be dependent on a variety of factors including, e.g. infectious dose, virus type, infection pathway, and the like, In cancer treatment, treating preferably comprises inhibiting infiltration and/or metastasis. Treating cancer may, however, also comprise reducing tumor and/or cancer cell burden in a subject, e.g. by prevention of neovascularization. As will be understood by the skilled person, effectiveness of treatment of e.g. cancer is dependent on a variety of factors including, e.g. cancer stage and cancer type.

The terms "preventing" and "prevention" refer to retaining health with respect to the diseases or disorders referred to herein for a certain period of time in a subject. It will be understood that the said period of time may be dependent on the amount of the drug compound which has been administered and individual factors of the subject discussed elsewhere in this specification. It is to be understood that prevention may not be effective in all subjects treated with the compound according to the present invention. However, the term requires that, preferably, a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification. In the context of cancer treatment, preventing in particular relates to preventing infiltration, preventing metastasis formation, and/or preventing relapse. In the context of virus infection, prevention may in particular be preventing virus entry into target cells, e.g. of inhaled virus particles.

The term "subject", as referred to herein, relates to a vertebrate animal, preferably a mammal, in particular a livestock, companion, or laboratory animal. More preferably, subject is a human. Preferably, the subject is known or suspected to suffer from a disease referred to herein, is known or suspected to be at risk of suffering from such disease, e.g. virus infection, and/or is known or suspected to be at risk of suffering severe complications of such disease, such as in an immunocompromised subject.

The inhibitory polypeptide is preferably comprised in a pharmaceutical composition.

As used herein, the term "pharmaceutical composition", for which also the term "medicament" may be used, relates to a composition comprising at least the inhibitory polypeptide and optionally one or more pharmaceutically acceptable carriers, i.e. excipient. The inhibitory polypeptide can be formulated as a pharmaceutically acceptable salt as specified elsewhere herein. The pharmaceutical composition is preferably prepared by combining the inhibitory polypeptide with standard pharmaceutical carriers according to conventional procedures. These procedures may involve mixing, granulating and compressing or dissolving the ingredients as appropriate to obtain the desired pharmaceutical composition. It will be appreciated that the form and character of the pharmaceutically acceptable carrier or diluent is dictated by the amount of inhibitory polypeptide with which it is to be combined, the route of administration and other well-known variables. The excipient(s) must be acceptable in the sense of being compatible with the other ingredients of the formulation and being not deleterious to the recipient thereof. The excipient employed may be, for example, a solid, a gel or a liquid carrier. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania. The diluent(s) is/are preferably selected so as not to affect the biological activity of the inhibitory polypeptide. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, non-immunogenic stabilizers and the like. Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent, or enclosed or encapsulated in a capsule, sachet, cachet, paper or other suitable containers or vehicles. The resulting formulations are to be adapted to the mode of administration, i.e. in the forms of tablets, capsules, suppositories, solutions, suspensions, sprays or powders for inhalation, patches, or the like and can have modified release and protective characteristics (e.g. slow release, enteric coating). Dosage recommendations shall be indicated in the prescribers or users instructions in order to anticipate dose adjustments depending on the considered recipient.

As used herein, the term "pharmaceutically acceptable salt" may be understood in the broadest sense as any composition of matter comprising a charged form of the inhibitory polypeptide of the present description and at least one counterion. Depending on the chemical structure of the compound and on the environment it is dissolved in, the compound may, exemplarily, comprise one or more charged residue(s) selected from the group consisting of but not limited to carboxylate anion residue(s), primary ammonium cation(s), and secondary ammonium cation residue(s). The counterions may be any ions known to be pharmaceutically acceptable in the art such as, e.g., acetate ions, chloride ions, sodium ions, potassium ions, magnesium ions, calcium ions, aluminum ions, lithium ions, ammonium ions, phosphate ions, hydroxyl ions, protons and fluoride ions.

The inhibitory polypeptide or the pharmaceutical composition are, preferably, administered locally, topically or systemically, preferably are administered systemically. Suitable routes of administration of the compound or the pharmaceutical composition are inhalation, oral, intravenous, intramuscular, subcutaneous, rectal, transdermal, as well as parenteral administration. Preferably, the inhibitory polypeptide or the pharmaceutical composition are administered by inhalation, intravenously, or orally. However, depending on the desired speed of action of a compound, the pharmaceutical composition may be administered by other routes as well.

A therapeutically effective dose refers to an amount of the compounds to be used in a pharmaceutical composition which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50 % of the population) and LD50 (the dose lethal to 50 % of the population). The dose ratio between therapeutic and toxic effects is the therapeutic index, and it can be expressed as the ratio, LD50/ED50. The dosage regimen will be determined by the attending physician based on clinical factors; preferably in accordance with any one of the above described methods. As is well known in the medical arts, dosages for any one patient depends upon many factors, including disease severity and duration, success of previous therapies, the patient's size, body surface area, age, the particular compound to be administered, sex, genetics and genomics, differences in target expression (e.g. protein expression), time and route of administration, general health, food, co-morbidity, and other drugs being administered concurrently. Progress can be monitored by periodic assessment. A typical dose can be, for example, in the range of 0.1 to 1000 mg for a compound as referred to herein; however, doses below or above this exemplary range are envisioned, especially considering the aforementioned factors. The compound or pharmaceutical composition and formulations referred to herein are administered at least once in order to treat or prevent a disease or condition recited in this specification. However, the compound or pharmaceutical composition may be administered more than one time, for example from one to four times daily or continuously and up to a non-limited number of days.

Advantageously, it was found in the work underlying the present invention that the inhibitory polypeptides described herein inhibit transmembrane serine proteases and neutrophil elastase and therefore are useable in prevention and treatment of diseases associated with the activity of one of said proteases.

The definitions made above apply mutatis mutandis to the following. Additional definitions and explanations made further below also apply for all embodiments described in this specification mutatis mutandis.

The present invention further relates to a polynucleotide encoding the inhibitory polypeptide as specified herein for use in treating and/or preventing a TMPRSS and/or neutrophil elastase mediated disease.

The term "polynucleotide", has been specified herein above. The polynucleotide has the activity of encoding the inhibitory polypeptide as specified herein. Based on the amino acid sequences provided herein, the skilled person can provide appropriate polynucleotides encoding inhibitory polypeptides without further ado. In view of the redundancy of the genetic code, a plurality of polynucleotides encode the same inhibitory polypeptide may be envisaged. The polynucleotide may, in addition to the nucleic acid sequences encoding the aforementioned inhibitory polypeptide, comprise additional sequences required for proper transcription and/or translation such as 5'- or 3'-UTR sequences or sequences required for splicing or RNA stability. The polynucleotide shall be provided, preferably, either as an isolated polynucleotide (i.e. purified or at least isolated from its natural context such as its natural gene locus) or in genetically modified or recombinantly (i.e. artificially) manipulated form.

Preferably, the polynucleotide is comprised in an expression construct allowing for expression of the polynucleotide in a host cell or a subject.

The term "expression construct", as used herein, refers to a heterologous polynucleotide comprising the aforementioned polynucleotide encoding the inhibitory polypeptide as well as one or more nucleic acids being heterologous thereto which are required for expression of the polynucleotide encoding the fusion polypeptide. Typically, such heterologous nucleic acids may be promoter sequences, enhancer sequences and/or transcription termination sequences such as terminators. Moreover, in the expression construct, the polynucleotide is usually operatively linked to expression control sequences allowing expression in prokaryotic or eukaryotic host cells or isolated fractions thereof. Expression of the polynucleotide may comprise transcription of the polynucleotide into a translatable mRNA. Regulatory elements ensuring expression in host cells are well known in the art. Preferably, they comprise regulatory sequences ensuring initiation of transcription and/or poly-A signals ensuring termination of transcription and stabilization of the transcript. Regulatory sequences active in mammalian and other animal cells are known in the art. Other expression systems envisaged by the invention shall permit expression in insect cells, such as polyhedrin promoter-based systems. Moreover, inducible expression control sequences may be used, which are well known in the art. Beside elements which are responsible for the initiation of transcription such regulatory elements may also comprise transcription termination signals, such as the SV40-poly-A site or the tk-poly-A site, downstream of the polynucleotide.

Preferably, the expression construct and/or the polynucleotide referred to herein is a vector. A "vector", as referred to herein, preferably, encompasses phage, plasmid, viral or retroviral vectors as well as artificial chromosomes, such as bacterial or yeast artificial chromosomes. The vector encompassing the polynucleotide encoding the inhibitory polypeptide, preferably, further comprises selectable markers for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerens. Alternatively, a plasmid vector may be introduced by heat shock or electroporation techniques. Should the vector be a virus, it may be packaged in vitro using an appropriate packaging cell line prior to application to host cells. Viral vectors may be replication competent or replication defective. In the latter case, viral propagation generally will occur only in complementing host/cells. Moreover, the vector may also comprise further nucleic acids required for introducing the vector into a host. For example, if maintenance in host cells is desired, the vector may comprise further nucleic acids required for transformation or transfection and for propagation of the vector in the host cells. Preferably, the vector is an expression vector and a gene transfer or targeting vector. In this context, suitable expression vectors are known in the art.

The present invention further relates to a host cell releasing an inhibitory polypeptide as specified herein, wherein said host cell preferably comprises an expression construct and/or vector as specified herein.

The term "cell" is understood by the skilled person and preferably relates to any bacterial, archeal, or eukaryotic cell. Preferably, the cell is a living cell. The term "target cell" is used herein to relate to any cell being the aim of an action or of an event. Thus, a cell of a mucous membrane of a subject may be a target cell of a virus infection, a cancer cell may be a target cell of a chemotherapeutic compound, and the like; thus, the target cell preferably is a eukaryotic cell, preferably a mammalian cell, more preferably a human cell. Most preferably, the target cell is a cell of a subject as specified herein above. The target cell may, however, also be a cell cultured in vitro, The term "host cell", as used herein, relates to any cell capable of receiving and/or producing an inhibitory polypeptide and/or receiving and integrating or stably replicating a polynucleotide and/or expressing an expressible construct, both as specified herein above. Preferably, the host cell is a eukaryotic cell, preferably a plant or yeast cell, e.g. a cell of a strain of baker's yeast, or is an animal cell. More preferably, the host cell is an insect cell or a mammalian cell, in particular a mouse or rat cell. Even more preferably, the host cell is a mammalian cell, most preferably is a human cell. The host cell may, however, also be a bacterial cell, e.g. of a known human commensal strain, preferably of the respiratory tract.

The host cell has been described herein above. Preferably, the host cell releases an inhibitory polypeptide as specified herein, wherein the term "release" of a polypeptide relates to the activity of making the inhibitory polypeptide available in the medium surrounding the host cell.

Thus, releasing the inhibitory polypeptide may in particular be secreting the inhibitory polypeptide.

The present invention also relates to a composition comprising the inhibitory polypeptide as specified herein for use in treating and/or preventing a TMPRSS and/or neutrophil elastase mediated disease.

The term "composition", as used herein, relates to any composition of matter comprising at least an inhibitory polypeptide as specified herein and, preferably, at least one carrier. The composition may have any consistency deemed appropriate by the skilled person. Preferably, the composition is a solid composition, e.g. a tablet or a powder, a semisolid composition, e.g. a gel, or, more preferably, a liquid, e.g. a solution or an emulsion. The composition may comprise any further compound deemed appropriate by the skilled person, e.g. at least one further antiviral compound. Preferably, the composition is a pharmaceutical composition as specified herein above.

The present invention also relates to a use of an inhibitory polypeptide as specified herein and/or a composition as specified herein for the manufacture of a medicament for treating and/or preventing a TMPRSS and/or neutrophil elastase mediated disease.

The present invention also relates to a method for treating and/or preventing a TMPRSS and/or neutrophil elastase mediated disease in a subject, said method comprising contacting said subject with an inhibitory polypeptide as specified herein. The present invention also relates to a method for treating and/or preventing an infection with a respiratory virus in a subject, said method comprising contacting said subject with an inhibitory polypeptide as specified herein.

The methods of treatment, preferably, are in vivo methods. Moreover, they may comprise steps in addition to those explicitly mentioned above. For example, further steps may relate, e.g., to diagnosing disease before treatment, disease monitoring, and the like. Moreover, one or more of the method steps may be assisted or performed by automated equipment.

The present invention moreover relates to a method of inhibiting a TMPRSS and/or neutrophil elastase, said method comprising contacting said TMPRSS and/or neutrophil elastase with an inhibitory polypeptide as specified herein.

The method of inhibiting may be an in vivo method, e.g. as part of a method of treatment described herein above. Preferably, however, the method of inhibiting is an in vitro method. Moreover, the method of inhibiting may comprise steps in addition to those explicitly mentioned above. Moreover, one or more of the method steps may be assisted or performed by automated equipment, e.g. in a high-throughput facility.

The present invention also relates to a device comprising the inhibitory polypeptide as specified herein, wherein said device is a device configured for administration of said inhibitory polypeptide to the respiratory tract of a subject.

The term "device", as used herein, relates to a system of means comprising at least the means operatively linked to each other as to allow administration of said inhibitory polypeptide to the respiratory tract of a subject. Preferred means for such administration are well known in the art. How to link the means in an operating manner will depend on the type of means included into the device and on the specific kind of administration envisaged. Preferably, the means are comprised by a single device in such a case. Said device may accordingly include a delivery unit for the administration of the compound or composition and a storage unit for storing said compound or composition until administration. However, it is also contemplated that the means of the current invention may appear as separate devices and are, preferably, packaged together as a kit. The person skilled in the art will realize how to link the means without further ado. Preferred devices are those which can be applied without the particular knowledge of a specialized technician. Preferably, the device is an endoscopic device comprising the inhibitory polypeptide or medicament for flushing a site of administration, or further comprising a needle for topical application of the compound or composition. Also preferably, the device is an inhaler or nebulizer comprising the compound of the present invention, wherein, more preferably, said compound is formulated for administration as an aerosol.

Moreover, the present invention relates to a kit comprising the inhibitory polypeptide as specified herein, and a means for administration of said inhibitory polypeptide to the respiratory tract of a subject.

The term "kit", as used herein, refers to a collection of the aforementioned compounds, means or reagents. The components of the kit may be comprised by separate entities (i.e. as a kit of separate parts) or provided as a single entity. The kit may comprise a housing, wherein the housing of the kit preferably allows translocation of the compounds of the kit, in particular common translocation; thus, the housing may in particular be a transportable container comprising all specified components. Moreover, it is to be understood that the kit may be used for practicing at least one of the methods and/or treatments referred to herein above. It is envisaged that all components may be provided in a ready-to-use manner for practicing a method referred to above. Further, the kit preferably contains instructions for carrying out said methods, e.g. dosage instructions. The instructions can be provided by a user manual in paper- or electronic form. The kit preferably comprises further components, preferably at least one further antiviral compound as specified herein above, an excipient, and the like.

The present invention also relates to a use of an inhibitory polypeptide as specified herein or a composition as specified herein for preventing a virus infection, wherein said use preferably is an in vitro use.

In view of the above, the following embodiments are particularly envisaged:
Embodiment 1: An inhibitory polypeptide for use in treating and/or preventing a transmembrane serine protease (TMPRSS), preferably TMPRSS2, TMPRSS11D, TMPRSS13, and/or neutrophil elastase mediated disease in a subject, wherein said inhibitory polypeptide comprises an amino acid sequence of SEQ ID NO:1 or an amino acid sequence at least 70% identical thereto.
Embodiment 2: The inhibitory polypeptide for use of embodiment 1, wherein said inhibitory polypeptide comprises, preferably is, a trypstatin or a fragment thereof.
Embodiment 3: The inhibitory polypeptide for use of embodiment 1 or 2, wherein said inhibitory polypeptide has the activity of inhibiting TMPRSS and/or neutrophil elastase activity.
Embodiment 4: The inhibitory polypeptide for use of any one of embodiments 1 to 3, wherein said inhibitory polypeptide comprises an amino acid sequence of SEQ ID NO:1.
Embodiment 5: The inhibitory polypeptide for use of any one of embodiments 1 to 4, wherein said inhibitory polypeptide consists of an amino acid sequence of SEQ ID NO:1 or 2 or an amino acid sequence at least 70% identical thereto.
Embodiment 6: The inhibitory polypeptide for use of any one of embodiments 1 to 5, wherein said inhibitory polypeptide consists of an amino acid sequence of SEQ ID NO:1 or 2.
Embodiment 7: The inhibitory polypeptide for use of any one of embodiments 1 to 6, wherein said TMPRSS is TMPRSS2, TMPRSS11D, and/or TMPRSS13.
Embodiment 8: The inhibitory polypeptide for use of any one of embodiments 1 to 7, wherein said TMPRSS is human TMPRS2, preferably human TMPRS2 comprising the amino acid sequence of Genbank Acc No. NP_001128571.1, or is human TMPRS11D, preferably human TMPRS11D comprising the amino acid sequence of Genbank Acc No. NP_004253.1, or is human TMPRS13, preferably human TMPRS13 comprising the amino acid sequence of Genbank Acc No. NP_001070731.1, and/or wherein said neutrophil elastase is human neutrophil elastase, preferably human neutrophil elastase comprising the amino acid sequence of Genbank Acc No. NP001963.1.
Embodiment 9: The inhibitory polypeptide for use of any one of embodiments 1 to 8, wherein said TMPRSS and/or neutrophil elastase mediated disease is a virus infection, cancer, or pancreatitis, wherein said cancer preferably is prostate cancer.
Embodiment 10: The inhibitory polypeptide for use of any one of embodiments 1 to 9, wherein said virus infection is an infection with a respiratory virus.
Embodiment 11: The inhibitory polypeptide for use of embodiment 10, wherein said infection with a respiratory virus is a coronavirus infection, an influenza A infection, an influenza B infection, a human metapneumovirus infection, or a human parainfluenza virus infection.
Embodiment 12: The inhibitory polypeptide for use of any one of embodiments 1 to 11, wherein said TMPRSS and/or neutrophil elastase mediated disease is an infection with a SARS virus, with a MERS-CoV, with a hCoV-229E, with a hCoV-NL63, with a hCoV-OC43, or with a hCoV-HKU1.
Embodiment 13: The inhibitory polypeptide for use of any one of embodiments 1 to 12, wherein said TMPRSS and/or neutrophil elastase mediated disease is a SARS-CoV-2 infection.
Embodiment 14: The inhibitory polypeptide for use of any one of embodiments 1 to 13, wherein said inhibitory polypeptide is comprised in a pharmaceutical composition.
Embodiment 15: The inhibitory polypeptide for use of embodiment 14, wherein said subject is a mammal, preferably is a human.
Embodiment 16: A polynucleotide encoding the inhibitory polypeptide as specified in any one of embodiments 1 to 6 for use in treating and/or preventing a TMPRSS and/or neutrophil elastase mediated disease.
Embodiment 17: A host cell releasing the inhibitory polypeptide as specified in any one of embodiments 1 to 6 for use in treating and/or preventing a TMPRSS and/or neutrophil elastase mediated disease.
Embodiment 18: A composition comprising the inhibitory polypeptide as specified any one of embodiments 1 to 6 for use in treating and/or preventing a TMPRSS and/or neutrophil elastase mediated disease.
Embodiment 19: The composition for use of embodiment 18, wherein said composition further comprises a carrier.
Embodiment 20: The composition for use of embodiment 18 or 19, further comprising at least one further antiviral compound.
Embodiment 21: The composition for use of any one of embodiments 18 to 20, wherein said carrier is a pharmaceutically acceptable carrier.
Embodiment 22: The composition of any one of embodiments 18 to 21, wherein said composition is a pharmaceutical composition.
Embodiment 23: Use of an inhibitory polypeptide as specified in any one of embodiments 1 to 6 and/or a composition according to any one of embodiments 18 to 22 for the manufacture of a medicament for treating and/or preventing a TMPRSS and/or neutrophil elastase mediated disease.
Embodiment 24: A method for treating and/or preventing a TMPRSS and/or neutrophil elastase mediated disease in a subject, said method comprising contacting said subject with an inhibitory polypeptide as specified in any one of embodiments 1 to 6.
Embodiment 25: A method of inhibiting a TMPRSS and/or neutrophil elastase, said method comprising contacting said TMPRSS and/or neutrophil elastase with an inhibitory polypeptide as specified in any one of embodiments 1 to 6.
Embodiment 26: The method of embodiment 25, wherein said method is an in vitro method.
Embodiment 27: A device comprising the inhibitory polypeptides specified in any one of embodiments 1 to 6, wherein said device is a device configured for administration of said inhibitory polypeptide to the respiratory tract of a subject.
Embodiment 28: A kit comprising the inhibitory polypeptide as specified in any one of embodiments 1 to 6, and a means for administration of said inhibitory polypeptide to the respiratory tract of a subject.
Embodiment 29: The kit of embodiment 28, further comprising at least one further antiviral compound.
Embodiment 30: Use of an inhibitory polypeptide as specified in any one of embodiments 1 to 6 or a composition according to any one of embodiments 18 to 22 for preventing a virus infection.
Embodiment 31: The use of embodiment 30, wherein said use is an in vitro use.
Embodiment 32: An inhibitory polypeptide for use in treating and/or preventing a respiratory virus infection in a subject, wherein said inhibitory polypeptide comprises an amino acid sequence of SEQ ID NO:1 or an amino acid sequence at least 70% identical thereto.
Embodiment 33: The inhibitory polypeptide for use of embodiment 32, wherein said inhibitory polypeptide is an inhibitory polypeptide as specified in any one of embodiments 1 to 6.
Embodiment 34: The inhibitory polypeptide for use of embodiment 32 or 33, wherein said respiratory virus infection has a feature of any one of embodiments 11 to 13.

All references cited in this specification are herewith incorporated by reference with respect to their entire disclosure content and the disclosure content specifically mentioned in this specification.

### Figure Legends

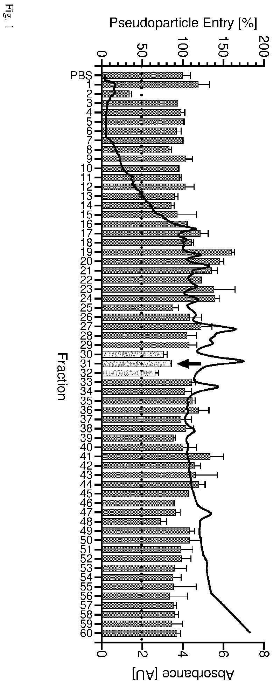

Fig. 1: Identification of a Trypstatin-like sequence from a hemofiltrate peptide/protein library screen. Screening for SARS-CoV-2 inhibitors. CaCo-2 cells were pre-treated with peptide/protein containing fractions of a hemofiltrate library before transduction with luciferase-encoding lentiviral pseudoparticles harboring the Wuhan Hu-1 spike. Transduction rates were assessed 48 h later by measuring luciferase activity in cell lysates. Grey columns represent pseudoparticle entry and black line absorbance at 280 nm of the corresponding fraction. Hit fractions 30-32 were subjected to mass spectrometry analysis and revealed high abundancy of a trypstatin-like sequence. Shown are mean values of one experiment performed in triplicates ± SEM.

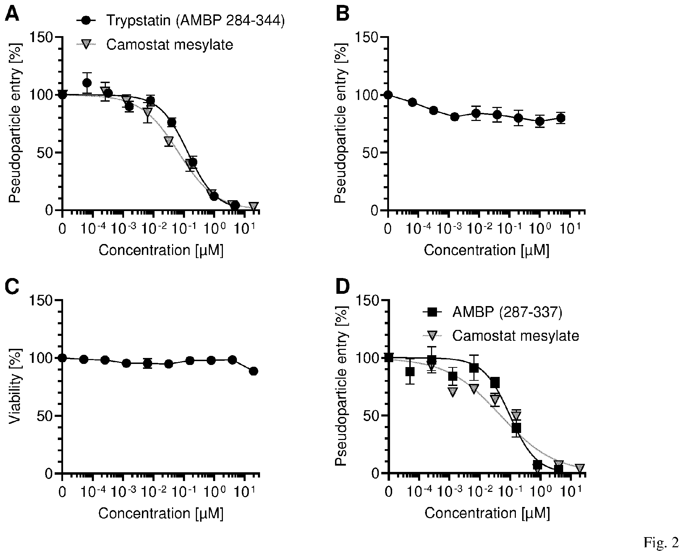

Fig. 2: Trypstatin inhibits SARS-CoV-2 spike but not VSV-G mediated pseudovirus entry. (A) CaCo-2 cells were pre-treated with serial dilution of Trypstatin (AMBP 284-344) or control inhibitor Camostat mesylate before transduction with luciferase-encoding lentiviral pseudoparticles harboring the Wuhan Hu-1 spike. Transduction rates were assessed 48 h later by measuring luciferase activity in cell lysates. (B) CaCo-2 cells were pre-treated with serial dilutions of Trypstatin before transduction with luciferase encoding lentiviral pseudoparticles harboring VSV-G glycoprotein. Transduction rates were assessed 48 h later by measuring luciferase activity in cell lysates. (C) CaCo-2 cells were treated with serial dilutions of Trypstatin for 48 h before cell viability was assessed using the Promega CellTiter-Glo^{®} Cell Viability Assay. (D) CaCo-2 cells were pre-treated with serial dilutions of AMBP 287-337 before transduction with luciferase encoding lentiviral pseudoparticles harboring the Wuhan Hu-1 spike glycoprotein. Transduction rates were assessed 48 h later by measuring luciferase activity in cell lysates. Shown are mean values of three independent experiments performed in triplicates ± SEM.

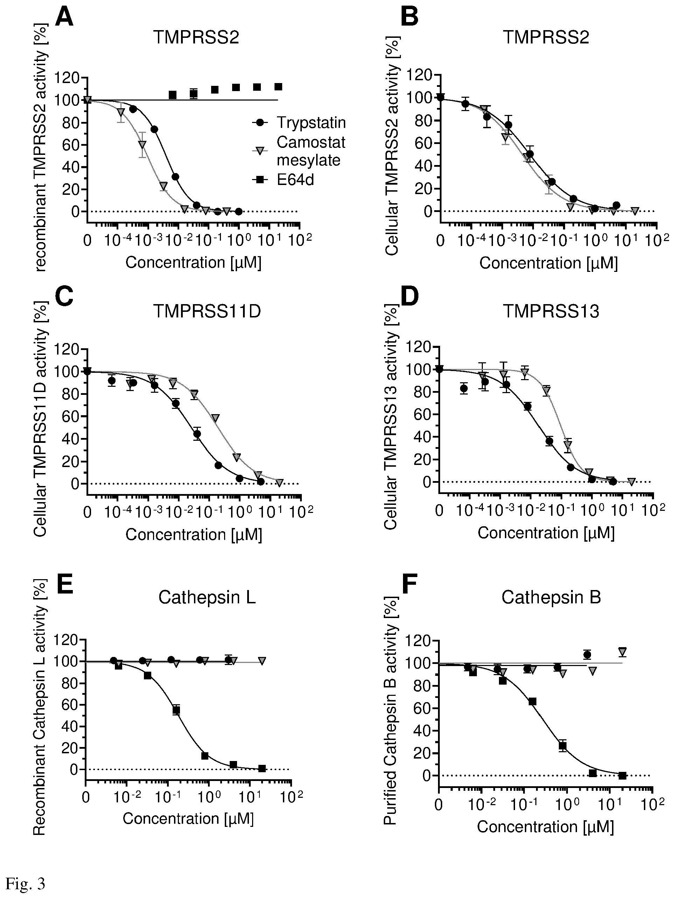

Fig. 3: Trypstatin inhibits type II transmembrane serine proteases (TMPRSS) but not cathepsins. (A, E-F) Serial dilutions of Trypstatin, serine protease inhibitor Camostat mesylate and cysteine protease inhibitor E64-d were mixed with respective enzyme before addition of a fluorogenic reporter substrate. Fluorescence intensity was measured at an excitation wavelength of 380 nm and emission wavelength of 460 nm. (B-D) HEK293T-cells were transfected with TMPRSS2-, TMPRSS11D- and TMPRSS13-encoding plasmid or mock control, respectively, before treatment with serial dilutions of inhibitors and the addition of a fluorogenic reporter substrate. Values were corrected for the signal of non-transfected HEK293T-cells. Shown are mean values of three independent experiments performed in triplicates ± SEM.

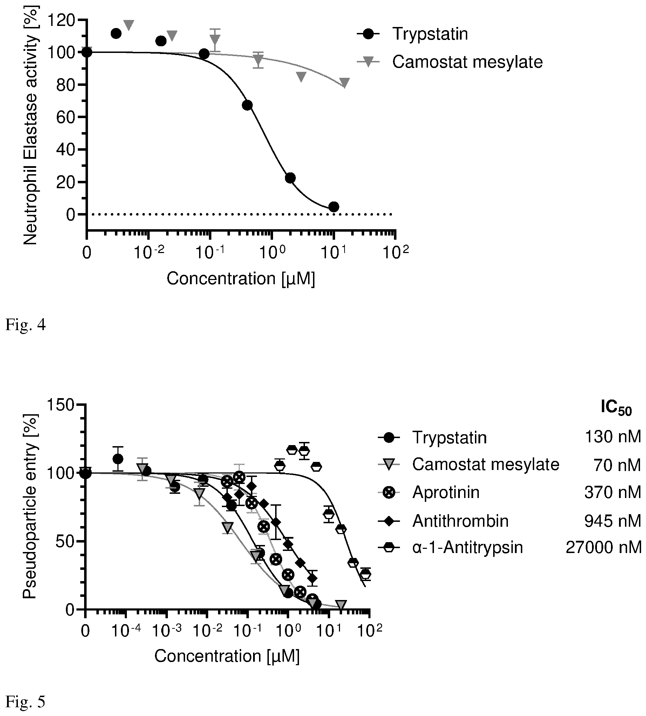

Fig. 4: Trypstatin inhibits Neutrophil Elastase activity. Serial dilutions of Trypstatin and serine protease inhibitor Camostat mesylate were mixed with neutrophil elastase before addition of a fluorogenic reporter substrate. Fluorescence intensity was measured at an excitation wavelength of 380 nm and emission wavelength of 460 nm. Shown are mean values of one experiment performed in triplicates ± SEM.
Fig. 5: Trypstatin surpasses the potency of currently approved protease inhibitors. CaCo-2 cells were pre-treated with serial dilution of compounds before transduction with luciferase-encoding lentiviral pseudoparticles harboring the SARS-CoV-2 Wuhan Hu-1 spike. Transduction rates were assessed 48 h later by measuring luciferase activity in cell lysates. Shown is the mean of one experiment performed in triplicates ± SEM.

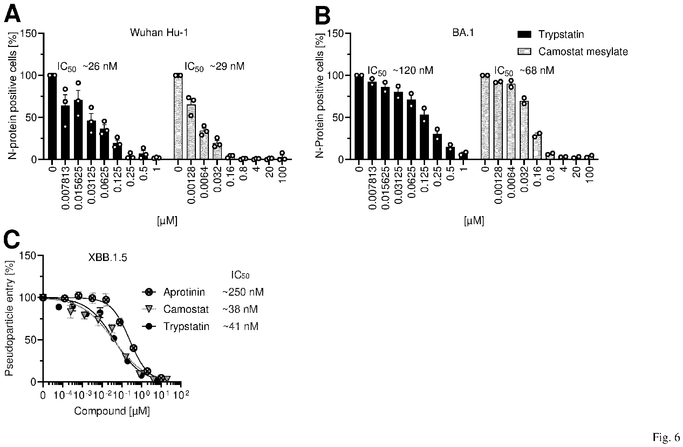

Fig. 6: Trypstatin inhibits authentic SARS-CoV-2 including the Omicron variant. (A) Trypstatin inhibits authentic SARS-CoV-2. CaCo-2 cells were treated with indicated compounds for 1h before inoculation with SARS-CoV-2 Hu-1 (MOI 0.0005) or the Omicron BA.1 variant (MOI 0.2). Nucleocapsid protein positive cells were quantified one day post infection by flow cytometry. (B) Trypstatin inhibits spike-driven entry of Omicron XBB.1.5. CaCo-2 cells were pre-treated with serial dilutions of Trypstatin, Camostat mesylate or Aprotinin before transduction with luciferase-encoding lentiviral pseudoparticles harboring the Omicron XBB.1.5 spike. Transduction rates were assessed 48 h later by measuring luciferase activity in cell lysates. Shown is the mean of two (A) or three (B-C) experiments performed in triplicates ± SEM.

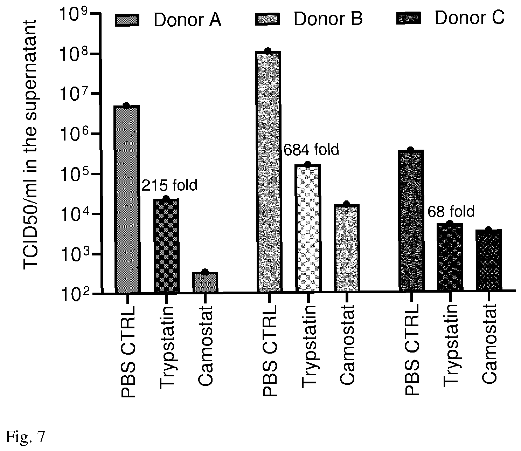

Fig. 7: Trypstatin inhibits replication of SARS-CoV-2 BA.5 in human air-liquid interface cultures (ALI cultures). The apical site of human airway epithelial cells (HAEC) grown at air-liquid interface was exposed to PBS, Trypstatin (10 µM) or Camostat mesylate (80 µM) before inoculation with SARS-CoV-2 BA. 5 (MOI 0.5) for 2 h before apical washing and further culturing at the air-liquid interface. Two days-post infection, mucus was washed off and PBS was added to the apical side for 30 minutes before the sample was subjected to TCID50 analysis.

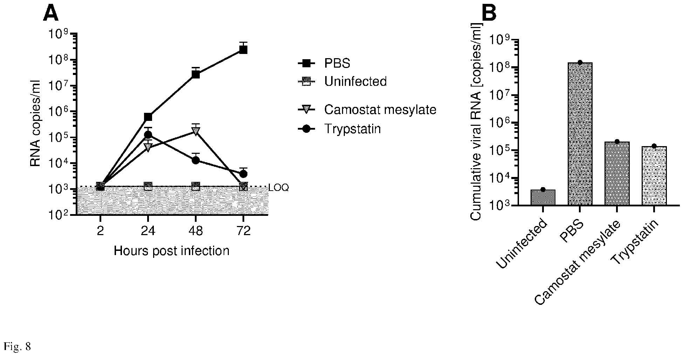

Fig. 8: Trypstatin reduces replication of SARS-CoV-2 BA.5 in human lung cells. Calu-3 cells were treated with Trypstatin (10 µM), Camostat (80 µM) or PBS right before infection with SARS-CoV-2 BA.5 at a MOI of 0.001. 2 h later, the inoculum was removed, cells were washed and fresh medium supplemented with respective compounds was added. Complete supernatants were sampled at 2, 24, 48, and 72 h postinfection (hpi) and fresh medium with compound added after sampling. (A) RT-qPCR targeting SARS-CoV-2 ORFlb nsp 14 of harvested supernatants. (B) Cumulative SARS-CoV-2 RNA copies until 3 dpi assessed by area under the curve analysis from (A).

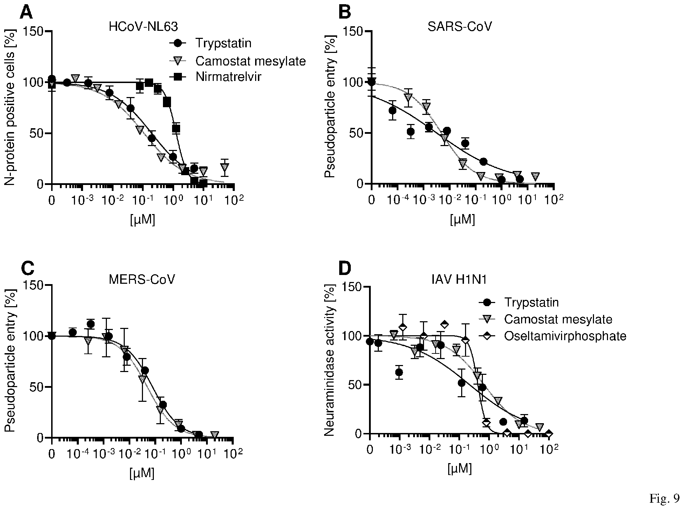

Fig. 9: Trypstatin broadly inhibits TMPRSS2-dependent viruses. (A-C) Trypstatin is a broad coronavirus inhibitor. (A) CaCo-2 cells were treated with serial dilutions of Trypstatin or control inhibitors before infection with hCoV-NL63 wt-virus. Nucleocapsid protein positive cells were quantified three days post infection by flow cytometry. (B-C) CaCo-2 cells were treated with serial dilutions of compound before transduction with lentiviral pseudoparticles harboring the SARS- or MERS-CoV spike. Transduction rates were assessed 48 h later by measuring luciferase activity in cell lysates. (D) CaCo-2 cells were treated with compounds before infection with IAV/PR-8/H1N1 virus. Viral titer in supernatant was measured by quantification of Neuraminidase activity in supernatant. Shown is the mean of three (A-C) or one (D) experiments performed in triplicates ± SEM.

The following Examples shall merely illustrate the invention. They shall not be construed, whatsoever, to limit the scope of the invention.

### Example 1: Material and Methods

1.1 Cell culture: CaCo-2 cells were grown in DMEM supplemented with in 100 U/ml penicillin, 100 µg/ml streptomycin, 1 mM sodium pyruvate, 2 mM L-glutamine, 1x non-essential amino acids and 20 % fetal calf serum (FCS). HEK293T-cells were cultivated in DMEM supplemented with 100 U/ml penicillin, 100 µg/ml streptomycin, 2 mM L-glutamin and 10 % FCS. Calu-3 cells were cultured in Minimum Essential Medium Eagle (MEM) supplemented with 10 % heat-inactivated FCS, 100 units/ml penicillin, 100 µg/ml streptomycin, 1 mM sodium pyruvate and 1x non-essential amino acids. LLC-MK-2 cells were cultured in Minimum Essential Medium Eagle (MEM) supplemented with 8 % heat-inactivated FCS for culture and 2 % for infection, 100 units/ml penicillin, 100 µg/ml streptomycin, 2 mM L-glutamine and 1x non-essential amino acids. TMPRSS2-expressing Vero E6 cells were cultivated in DMEM supplemented with 10 % FCS, 2 mM L-glutamine, 100 U/ml penicillin, 100 mg/ml streptomycin and 1 mg/ml geneticin. All cells were cultivated at 37°C in a 5 % CO₂ humidified incubator. Cells were tested for mycoplasma contamination on a regular basis.

1.2 Production of lentiviral pseudoparticles: Lentiviral pseudoparticles were produced as described before [1].

1.3 Preparation of hemofiltrate library: Peptide/Protein library was generated as described [2].

1.4 Screening hemofiltrate library for inhibitors of SARS-CoV-2: Was performed as described previously [3].

1.5 Pseudoparticle inhibition assay: Assay was performed as described previously [1]. XBB.1.5 spike encoding plasmid (pCG1_SARS-2-SΔ18) was kindly provided by Stefan Pöhlmann, DPZ Göttingen.

1.6 Recombinant Enzyme inhibition assays: Inhibition assay for recombinant TMPRSS2, Cathepsin L, Cathepsin B and Neutrophil Elastase was performed as described previously [1].

1.7 Cellular TMPRSS inhibition assay: Assay was performed as described [1]. TMPRSS2, - 11D and -13 plasmids were obtained from Twist Bioscience.

1.8 Viral strains and propagation: HCoV-NL63 was obtained from Lia van der Hoek, Amsterdam University and propagated as described before [4], [5]. SARS-CoV-2 BA.1 (Omicron variant, lineage B.1.1.529, GSAID reference hCoV-19/Netherlands/NH-RIVM-71084/2021) was retrieved from European Virus Archive, Omicron BA.5 (B.1.1.529) was kindly provided by Prof. Dr. Florian Schmidt (University of Bonn), and propagated as described previously [1]. Influenza A strain A/PR/8/34 (H1N1, PR8) was purchased from ATCC (#VR-95) and propagated in MDCK cells as reported previously [6].

1.9 SARS-CoV-2 inhibition assay: SARS-CoV-2 inhibition assay in CaCo-2 and Calu-3 cells was performed as described before [1].

1.10 Generation of human airway epithelial cells (HAECs): Was performed as described previously [3].

1.11 SARS-CoV-2 infection of HAECs: Right before infection, the apical surface of HAECs grown on Transwell filters at the air-liquid-interface were washed three times with pre-heated PBS to remove mucus. Subsequently, 10 µM Trypstatin, 80 µM Camostat mesylate or PBS were added onto the apical surface for 10 minutes before viral inoculum (SARS-CoV-2 Omicron BA.5, MOI 0.5) was added for 2 hours. Following this, inoculum and compound was removed, cells washed with PBS and further cultured at the air-liquid interface. Accumulated mucus was washed off daily with pre-heated PBS. Two days post infection, mucus was removed and PBS added to the apical side for 30 minutes before the sample was subjected to TCID50 analysis on Vero E6 cells. TCID50 was quantified 6 days post infection.

1.12 HCoV-NL63 inhibition assay: Assay was performed as described previously [4].

1.13 Influenza A virus inhibition assay: For inhibition assay, 20,000 CaCo cells were seeded in 96-well plate in 100 µl medium. The next day, cells were washed two times with PBS before they were infected with a MOI of 0.1 of IAV PR8 in a total volume of 100 µl DMEM supplemented with 0.2 % bovine serum albumin (BSA), 2 mM L-glutamine, 100 U/ml penicillin and 100 mg/ml streptomycin and 25 mM HEPES buffer. After 1 h incubation at 37°C, cells were washed two times with PBS and was replaced with fresh growth medium and the respective compound of interest was added. After 48 hours, infectivity rates were determined by measuring neuraminidase activity in supernatants (MUNANA assay). To that end, supernatants were centrifuged for 5 min at 3000 rpm. 90 µl supernatant was lysed in 10 µl 10% Triton-X100 for 30 min. The lysates were diluted 1:2 in MES buffer (containing 32.5 mM MES monohydrate and 4 mM CaCl₂ dihydrate). 20 µl of each sample were transferred to black 96-well plates and 30 µl 10 µM 20-(4-methylumbelliferyl)-a-D-N-acetylneuraminic acid (MUNANA) were added. After 4 h of incubation at 37°C and gentle shaking, reaction was stopped with 150 µl stop solution containing 0.1 M glycine and 25% ethanol. Neuraminidase-dependent cleavage of the substrate to the fluorescent product methylumbelliferone was quantified at an excitation of 360 nm and an emission at 455 nm using a Synergy^{™} H1 (BioTek, USA) plate reader.

1.14 Cytotoxicity assay: Assay was performed as described previously [3].

1.15 Reagents: Camostat mesylate (#SML0057) and E-64d (#E8640) were obtained from Merck. Antithrombin (Anbinex^{®}) and α1-antitrypsin (Prolastin^{®}) were obtained from Grifols. Aprotinin was purchased from Sigma Aldrich (#A1153), Nirmatrelvir from Selleckchem (#PF-07321332) and Oseltamivirphosphate from Santa Cruz Biotechnology (#SC-208135).

1.16 Peptide synthesis and refolding: Trypstatin peptide sequence was retrieved from Uniprot (AMBP 284-344 (SEQ ID NO:2)/287-337 (SEQ ID NO:1), #P02760). Trypstatin as well as the shortened sequence (AMBP 287-337) was synthesized by Synpeptide Co. Ltd., Shanghai, China. Both peptides were refolded to introduce their disulfide bridges. In brief, 10 mg of peptide was dissolved in 4 ml of denaturing buffer (50 mM Tris pH 8.5, 157 mM NaCl, 6 M GuHCl) containing 48 µl of a 1 M DTT solution before the mixture was incubated for 40 minutes at 40°C at 600 rpm. Afterwards, 4 ml of denaturing buffer was added before 59.8 mg Glutathione disulfide (GSSG) was dissolved in the mixture and dialyzed against 500 ml of renaturing buffer (50 mM Tris pH 8.5, 157 mM NaCl) for 16 hours at room temperature with magnetic stirring. The refolded sample was subjected to reversed-phase HPLC on an Aeris PEPTIDE XB-C18 column (Phenomenex, USA) of dimensions 1 x 250 mm and particle size of 5 µm, heated at 37°C. The separation was performed at a flow rate of 3 mL/min using the gradient program (min/%B) 0/5, 5/15, 65/45, 75/80, being A, 0.1%TFA in water, and B, 0.1% TFA in acetonitrile. Elution was monitored online at 280 nm. An Agilent 1100 Series (Agilent, USA) chromatographic system was used for the separation. Fractions were collected every minute and dried in a vacuum concentrator system (LABCONCO, USA). Purification resulted in two peaks, where the dominant exhibited anti-trypsin activity. Subsequently, samples were analyzed by an Axima Confidence MALDI-TOF MS (Shimadzu, Japan) in positive linear mode on a 384-spot stainless-steel sample plate. Spots were coated with 1 µL 5 mg/mL CHCA previously dissolved in matrix diluent (Shimadzu, Japan), and the solvent was allowed to air dry. Then, a 0.5 µL sample or standard was applied onto the dry pre-coated well and immediately mixed with 0.5 µL matrix; the solvent was allowed to air dry. All spectra were acquired in the positive ion linear mode using a 337-nm N2 laser. Ions were accelerated from the source at 20 kV. A hundred profiles were acquired per sample, and 20 shots were accumulated per profile. The equipment was calibrated with a standard mixture in the TOFMixTM MALDI kit (Shimadzu, Japan). Measurements and MS data processing were controlled by the MALDI-MS Application Shimadzu Biotech Launchpad 2.9.8.1 (Shimadzu, Japan).

1.17 Matrix-assisted laser-desorption ionization time-of-flight mass spectrometry (MALDI-TOF-MS): The sample was reduced with 5 mM DTT for 20 min at RT, carbamidomethylated with 50 mM iodoacetamide for 20 min at 37°C, and quenched with 10 mM DTT. A 15 µL-aliquot was analyzed by a nanoLC-Orbitrap Elite Hybrid mass spectrometry system (Thermo Fisher Scientific, Bremen, Germany) as previously described [7]. Database searches were performed using PEAKs XPro (PEAKs studio 10.6) [8]. For peptide identification, MS/MS spectra were correlated with the UniProt human reference proteome set, www.uniprot.org. Carbamidomethylated cysteine was considered as a fixed modification along with oxidation (M) and deamidation (NQ) as variable modifications. False discovery rates were set on the peptide level to 1%.

1.18 Non-linear regression and statistics: Unless stated otherwise, analysis was performed using GraphPad Prism version 10.0.2. Calculation of IC50 values via nonlinear regression was performed using normalized response-variable slope equation.

### Example 2: Results

### 2.1 Identification of Trypstatin as a SARS-CoV-2 inhibitor from a hemofiltrate library screen

In search for new endogenous inhibitors of SARS-CoV-2, a hemofiltrate peptide/protein library was screened using lentiviral pseudoviruses harboring the SARS-CoV-2 Wuhan Hu-1 spike. Hit fractions 30-32 showed reduction in pseudovirus entry (Fig. 1) and were subjected to mass spectrometry analysis which revealed high abundancy of a sequence similar to the Trypstatin peptide (SEQ ID NO:2).

### 2.2 Trypstatin inhibits SARS-CoV-2 spike but not VSV-G-mediated pseudovirus entry

The Trypstatin peptide was synthesized and refolded before activity was confirmed by evaluating inhibitory activity against SARS-CoV-2 spike-driven entry. Remarkably, Trypstatin peptide demonstrated high potency in the nanomolar range (IC₅₀ ~170 nM) similar to small molecule inhibitor Camostat mesylate (Fig. 2A) without compromising cell viability (Fig. 2C). Furthermore, the effect showed specificity towards the SARS-CoV-2 Wuhan Hu-1 spike and did not affect entry of VSV-G bearing control pseudoparticles (Fig. 2B). Trypstatin peptide was also shortened to only contain the kunitz-type protease inhibitory domain of Trypstatin which resulted in similar activity compared to the full-length peptide (Fig. 2D), suggesting that this might be the minimal active sequence (Alpha-1-microglobulin/bikunin precursor, AMBP 287-337, SEQ ID NO:1).

### 2.3 Trypstatin inhibits SARS-CoV-2 spike-priming protease TMPRSS2

It was hypothesized that Trypstatin might act on one of the SARS-CoV-2 spike-priming proteases required for cell entry. To asses this, either recombinant transmembrane protease serine subtype 2 (TMPRSS2) or Cathepsin L/B was mixed with Trypstatin peptide before the addition of a fluorogenic reporter substrate. In a second approach, the respective TMPRSS protease was overexpressed on cells before addition of inhibitor and reporter substrate. This clearly unveiled, that Trypstatin inhibits the TMPRSS2 protease required for cell surface entry of SARS-CoV-2 but not Cathepsin L or B, enabling the endosomal entry of the virus( Fig. 3A-B; E-F) [9]. Intriguingly, Trypstatin also inhibited other transmembrane serine proteases (TMPRSS 11D and TMPRSS 13) that are reported to enable cell entry of SARS-CoV-2 and other respiratory viruses (Fig. 3C-D) [10]-[13], but did not inhibit Cathepsin L and B (Fig. 3 E-F).

### 2.4 Trypstatin inhibits Neutrophil Elastase

Besides the evaluation of the well-known proteases priming viral entry, it was also assessed whether the Trypstatin peptide inhibits neutrophil elastase activity. On the one hand, it was described, that elastase can enhance viral replication by cleaving viral glycoproteins [14], [15], on the other hand, elastase can be upregulated during inflammation and viral infection in the lungs and lead to tissue damage and emphysema, if aberrantly abundant [16]. As depicted in Figure 4, Trypstatin indeed lead to a dose-dependent inhibition of neutrophil elastase activity, hinting towards a potential tissue protecting ability in addition to the direct antiviral activity.

### 2.5 Trypstatin surpasses the potency of currently approved protease inhibitors

Subsequently, an analysis was conducted to evaluate the performance of other currently approved protease inhibitors, known to inhibit TMPRSS2 [1], [3], [9], [17] and consequently SARS-CoV-2 spike-driven entry, in comparison to Trypstatin. As depicted in Figure 5, Trypstatin showed similar activity (IC₅₀ 130 nM) to small molecule inhibitor Camostat mesylate (IC₅₀ 70 nM) and displayed lower IC₅₀ values compared to approved protease inhibitors Anithrombin (Anbinex^{®}), α-1-Antitrypsin (Prolastin^{®}) and Aprotinin (Trasylol^{®}) with IC₅₀ values of 945 nM, 27000 nM and 370 nM, respectively.

### 2.6 Trypstatin inhibits replication competent SARS-CoV-2

To support the findings based on the pseudovirus platform, experiments with authentic SARS-CoV-2 were performed. Quantification of N-protein expression one day post infection by flow cytometry confirmed that Trypstatin inhibits infection by SARS-CoV-2 Wuhan Hu-1 strain (Fig. 6A) but also the SARS-CoV-2 Omicron BA.1 variant with similar efficacy (IC₅₀ values of 26 nM and 120 nM, respectively) compared to the pseudovirus assays (Fig. 6B). In addition, the at the time of the experiment pre-dominant Omicron XBB.1.5 variant was tested in a pseudoparticle assay which verified activity of Trypstatin across different variants (Fig. 6C). To further strengthen the findings, experiments with human lung cells (Calu-3 cells) and primary human airway epithelial cells (HAECs) grown at the air-liquid interface were conducted. As shown in Figure 7, a single initial treatment with Trypstatin strongly reduced viral titers two days post infection with the Omicron BA.5 variant in all 3 donors tested. In line with these results, infection of human lung cells (Calu-3 cells) with daily sampling of supernatants over the course of three days revealed exponential growth in the PBS control from 10⁵ RNA copies/ml to almost 10⁹ RNA copies/ml while Trypstatin treatment almost entirely prevented replication until day 3 post infection (Fig. 8A). Analysis of cumulative RNA copy production demonstrated a more than >99.9 % reduction by Trypstatin (Fig. 8B). Thus, Trypstatin inhibits SARS-CoV-2 across variants in different cellular systems including human lung cells and primary human airway epithelial cells grown at the air-liquid interface.

### 2.7 Trypstatin broadly inhibits TMPRSS2-dependent viruses

Since SARS-CoV-2 is not the only virus hijacking TMPRSS2 for host cell entry[11]-[13], [18]-[20], other viruses were tested for susceptibility of TMPRSS2 inhibition by Trypstatin. Further corroborating the previous findings, Trypstatin was able to dose dependently block human coronavirus NL63 (hCoV-NL63, Fig. 9A, IC₅₀ 190 nM), the spike-driven entry of SARS-CoV (Fig. 9B, IC₅₀ 31 nM) and Middle east respiratory syndrome coronavirus (MERS-CoV, Fig. 9C, IC₅₀ 78 nM) as well as the replication of Influenza A virus (IAV) H1N1 (Fig. 9D, IC₅₀ 222 nM) in human cells. Remarkably, IC₅₀ values remained in the nanomolar range, similar to the results obtained previously for SARS-CoV-2. This demonstrates that Trypstatin is a broad inhibitor of viruses utilizing transmembrane serine proteases for entry into host cells.

References:
Belouzard et al. (2010) JBC 285(3):22758
Böttcher et al. (2006) JVI 80(19):9896
Emboriadou et al. (2007) Ann Clin Lab Sci 37(1):79
Hoffmann et al. (2020) Cell 181: 271-280.e8
Hoffmann et al. (2020), Antimicrob. Agents Chemother. 64: 19-21
Hoffmann et al. (2021) EbioMedicine 65:103255
Kishimoto et al. (2021) Viruses 13:384
Ko et al. (2020) Oncogene 39:5950
Redondo-Calvo et al. (2022) Eur J Clin Invest 52:e13776

### References from the Examples section

[1] L. Wettstein et al., "Native and activated antithrombin inhibits TMPRSS2 activity and SARS-CoV-2 infection," J. Med. Virol., vol. 95, no. 1, p. e28124, 2023, doi: 10.1002/jmv.28124.
[2] O. Zirafi et al., "Discovery and characterization of an endogenous CXCR4 antagonist," Cell Rep., vol. 11, no. 5, pp. 737-747, May 2015, doi: 10.1016/j.celrep.2015.03.061.
[3] L. Wettstein et al., "Alpha-1 antitrypsin inhibits TMPRSS2 protease activity and SARS-CoV-2 infection," Nat. Commun., vol. 12, no. 1, pp. 1-10, 2021, doi: 10.1038/s41467-021-21972-0.
[4] T. Weil, J. Lawrenz, A. Seidel, J. Münch, and J. A. Müller, "Immunodetection assays for the quantification of seasonal common cold coronaviruses OC43, NL63, or 229E infection confirm nirmatrelvir as broad coronavirus inhibitor," Antiviral Res., vol. 203, p. 105343, Jul. 2022, doi: 10.1016/j.antiviral.2022.105343.
[5] J. Lawrenz et al., "Severe Acute Respiratory Syndrome Coronavirus 2 Vaccination Boosts Neutralizing Activity Against Seasonal Human Coronaviruses," Clin. Infect. Dis. Off. Publ. Infect. Dis. Soc. Am., vol. 75, no. 1, pp. e653-e661, Aug. 2022, doi: 10.1093/cid/ciac057.
[6] K. M. J. Sparrer et al., "TRIM23 mediates virus-induced autophagy via activation of TBK1," Nat. Microbiol., vol. 2, no. 11, Art. no. 11, Nov. 2017, doi: 10.1038/s41564-017-0017-2.
[7] A. Rodriguez-Alfonso et al., "Advanced EPI-X4 Derivatives Covalently Bind Human Serum Albumin Resulting in Prolonged Plasma Stability," Int. J. Mol. Sci., vol. 23, no. 23, p. 15029, Nov. 2022, doi: 10.3390/ijms232315029.
[8] J. Zhang et al., "PEAKS DB: de novo sequencing assisted database search for sensitive and accurate peptide identification," Mol. Cell. Proteomics MCP, vol. 11, no. 4, p. M111.010587, Apr. 2012, doi: 10.1074/mcp.M111.010587.
[9] M. Hoffmann et al., "Camostat mesylate inhibits SARS-CoV-2 activation by TMPRSS2-related proteases and its metabolite GBPA exerts antiviral activity," EBioMedicine, vol. 65, p. 103255, Mar. 2021, doi: 10.1016/j.ebiom.2021.103255.
[10] M. Kishimoto et al., "TMPRSS11D and TMPRSS13 Activate the SARS-CoV-2 Spike Protein," Viruses, vol. 13, no. 3, p. 384, Feb. 2021, doi: 10.3390/v13030384.
[11] M. Abe et al., "TMPRSS2 Is an Activating Protease for Respiratory Parainfluenza Viruses," J. Virol., vol. 87, no. 21, pp. 11930-11935, Nov. 2013, doi: 10.1128/JVI.01490-13.
[12] Y. Shirogane et al., "Efficient Multiplication of Human Metapneumovirus in Vero Cells Expressing the Transmembrane Serine Protease TMPRSS2," J. Virol., vol. 82, no. 17, pp. 8942-8946, Sep. 2008, doi: 10.1128/JVI.00676-08.
[13] E. Böttcher, T. Matrosovich, M. Beyerle, H.-D. Klenk, W. Garten, and M. Matrosovich, "Proteolytic Activation of Influenza Viruses by Serine Proteases TMPRSS2 and HAT from Human Airway Epithelium," J. Virol., vol. 80, no. 19, pp. 9896-9898, Oct. 2006, doi: 10.1128/JVI.01118-06.
[14] S. Matsuyama, M. Ujike, S. Morikawa, M. Tashiro, and F. Taguchi, "Protease-mediated enhancement of severe acute respiratory syndrome coronavirus infection," Proc. Natl. Acad. Sci. U. S. A., vol. 102, no. 35, pp. 12543-12547, Aug. 2005, doi: 10.1073/pnas.0503203102.
[15] S. Belouzard, I. Madu, and G. R. Whittaker, "Elastase-mediated Activation of the Severe Acute Respiratory Syndrome Coronavirus Spike Protein at Discrete Sites within the S2 Domain," J. Biol. Chem., vol. 285, no. 30, pp. 22758-22763, Jul. 2010, doi: 10.1074/jbc.M110.103275.
[16] M. Emboriadou et al., "Human Neutrophil Elastase in RSV Bronchiolitis," Ann. Clin. Lab. Sci., vol. 37, no. 1, pp. 79-84, Dec. 2007.
[17] D. Bojkova et al., "Aprotinin Inhibits SARS-CoV-2 Replication," Cells, vol. 9, no. 11, Art. no. 11, Nov. 2020, doi: 10.3390/cells9112377.
[18] K. Shirato, M. Kawase, and S. Matsuyama, "Wild-type human coronaviruses prefer cell-surface TMPRSS2 to endosomal cathepsins for cell entry," Virology, vol. 517, pp. 9-15, Apr. 2018, doi: 10.1016/j.virol.2017.11.012.
[19] K. Shirato, M. Kawase, and S. Matsuyama, "Middle East Respiratory Syndrome Coronavirus Infection Mediated by the Transmembrane Serine Protease TMPRSS2," J. Virol., vol. 87, no. 23, pp. 12552-12561, Dec. 2013, doi: 10.1128/JVI.01890-13.
[20] L. M. Reinke et al., "Different residues in the SARS-CoV spike protein determine cleavage and activation by the host cell protease TMPRSS2," PloS One, vol. 12, no. 6, p. e0179177, 2017, doi: 10.1371/journal.pone.0179177.

## Claims

1. An inhibitory polypeptide for use in treating and/or preventing a transmembrane serine protease (TMPRSS) and/or neutrophil elastase mediated disease in a subject, wherein said inhibitory polypeptide comprises an amino acid sequence of SEQ ID NO:1 or an amino acid sequence at least 70% identical thereto.

2. The inhibitory polypeptide for use of claim 1, wherein said inhibitory polypeptide has the activity of inhibiting TMPRSS and/or neutrophil elastase activity.

3. The inhibitory polypeptide for use of claim 1 or 2, wherein said inhibitory polypeptide consists of an amino acid sequence of SEQ ID NO:1 or 2 or an amino acid sequence at least 70% identical thereto.

4. The inhibitory polypeptide for use of any one of claims 1 to 3, wherein said inhibitory polypeptide consists of an amino acid sequence of SEQ ID NO:1 or 2.

5. The inhibitory polypeptide for use of any one of claims 1 to 4, wherein said TMPRSS is TMPRSS2, TMPRSS11D, and/or TMPRSS13.

6. The inhibitory polypeptide for use of any one of claims 1 to 5, wherein said TMPRSS and/or neutrophil elastase mediated disease is a virus infection, cancer, or pancreatitis, wherein said cancer preferably is prostate cancer.

7. The inhibitory polypeptide for use of any one of claims 1 to 6, wherein said virus infection is an infection with a respiratory virus, preferably is a coronavirus infection, an influenza A infection, an influenza B infection, a human metapneumovirus infection, or a human parainfluenza virus infection.

8. The inhibitory polypeptide for use of any one of claims 1 to 7, wherein said TMPRSS and/or neutrophil elastase mediated disease is an infection with a SARS virus, with a MERS-CoV, with a hCoV-229E, with a hCoV-NL63, with a hCoV-OC43, or with a hCoV-HKU1.

9. The inhibitory polypeptide for use of any one of claims 1 to 8, wherein said TMPRSS and/or neutrophil elastase mediated disease is an infection with a SARS-CoV-2.

10. A polynucleotide encoding the inhibitory polypeptide as specified in any one of claims 1 to 6 for use in treating and/or preventing a TMPRSS and/or neutrophil elastase mediated disease.

11. A host cell releasing the inhibitory polypeptide as specified in any one of claims 1 to 4 for use in treating and/or preventing a TMPRSS and/or neutrophil elastase mediated disease.

12. A device comprising the inhibitory polypeptides specified in any one of claims 1 to 4, wherein said device is a device configured for administration of said inhibitory polypeptide to the respiratory tract of a subject.

13. A kit comprising the inhibitory polypeptide as specified in any one of claims 1 to 4, and a means for administration of said inhibitory polypeptide to the respiratory tract of a subject.

14. In vitro use of an inhibitory polypeptide as specified in any one of claims 1 to 4 for preventing a virus infection.

15. An inhibitory polypeptide for use in treating and/or preventing a respiratory virus infection in a subject, wherein said inhibitory polypeptide comprises an amino acid sequence of SEQ ID NO:1 or an amino acid sequence at least 70% identical thereto.
